# EUROPEAN PATENT APPLICATION

(11) **EP 1 462 108 A1**
(43) Date of publication of application: **29.09.2004**
(21) Application number: 02788576.3
(22) Date of filing: 17.10.2002
(51) Int. Cl.: A61K 35/00

(54) **INTERFERON G (G) PRODUCTION PROMOTER**

(30) Priority: 02.11.2001 JP 2001338508
(71) Applicant: Kabushiki Kaisha Hayashibara Seibutsu Kagaku Kenkyujo, Okayama-shi, Okayama 700-0907 (JP)
(72) Inventor: KUNIKATA, Toshio, Okayama-shi, Okayama 700-0907 (JP); ISHIHARA, Tatsuya, Okayama-shi, Okayama 700-0907 (JP); KURIMOTO, Masashi, Okayama-shi, Okayama 700-0907 (JP)
(74) Representative: Daniels, Jeffrey Nicholas
(86) International application number: PCT/JP2002/010804
(87) International publication number: WO 2003/037339

(57) **Abstract**

The object of the present invention is to provide an agent for promoting IFN-γ production, which can be safely applied to living bodies; the present invention solves the problem by providing an agent for promoting IFN-γ production, which comprises a tri-heterocyclic polymethine cyanine dye(s), and a method for producing IFN-γ, which comprises a step of allowing the agent to act on immunocompetent cells.

## Description

### TECHNICAL FIELD

The present invention relates to an agent for promoting interferon-γ production, more particularly, to a novel agent comprising a tri-heterocyclic polymethine cyanine dye as an effective ingredient for promoting interferon-γ production, and can be directly applied to living bodies.

### BACKGROUND ART

Interferon-γ (abbreviated as "IFN-γ", hereinafter) has been known as a protein having various biological activities such as anti-viral activity, anti-tumor activity, and immune regulatory activity. It is said to origin form immunocompetent cells stimulated with an antigen or mitogen. Due to having such biological activities, interferon-γ has been expected for practical use as an anti-viral agent or anti-tumor agent and is still earnestly studied for its clinical use as an agent'for treating malignant tumors including brain tumor, etc. INF-γ is usually classified into natural IFN-γ produced by immunocompetent cells and recombinant IFN-γ produced by *E. coli,* etc., transformed with IFN-γ genes, and either IFN-γ has been administrated to a patient as "extraneous IFN-γ" in the above clinical test.

Natural IFN-γ is usually prepared by culturing established immunocompetent cells in an appropriate culture medium containing an inducer for promoting IFN-γ production, and isolating the produced IFN-γ from the culture. The inducer must be very important for the production and purification of IFN-γ and safeness of its final product. Mitogens such as concanavalin A, lentil lectin, pokeweed mitogen, endotoxin, and lipopolysaccharide are usually and frequently used as the inducer. However, there is a problem that the above inducers having a constant activity for promoting IFN-γ, can not be prepared sufficiently because they have molecular polymorphism and easily change in quality depending on their sources and purification methods. In addition, there is another problem that most of them cannot be directly applied to living bodies to promote IFN-γ production because of their serious side effects and toxicity against the living bodies.

Under the above circumstance, the object of the present invention is to solve the above problems by providing an agent for promoting IFN-γ production, which can be safely applied to living bodies.

### DISCLOSURE OF THE INVENTION

The present inventors have eagerly studied polymethine cyanine dyes to attain the above object. As a result, they discovered that tri-heterocyclic polymethine cyanine dyes of polymethine cyanine dyes significantly promote INF-γ production by immunocompetent cells and does not have toxicity and serious side effects even when directly applied to living bodies.

The present invention solves the above problem by providing an agent comprising a tri-heterocyclic polymethine cyanine dye for promoting IFN-γ production, which.

The present invention solves the above problem by providing a method for producing IFN-γ which comprises the step of allowing the agent to act on immunocompetent cells.

Tri-heterocyclic polymethine cyanine dyes have been already disclosed, for example, in "KANKO-SHIKISO (PHOTOSENSITIZING DYE)", edited by Hayami, M., published by Sangyo Tosho Publishing Co., Ltd., pp.138-154, October 17, 1997, and used for treating anemia, anorexia, weak constitution, malaise, eczema, wound, burn injury, cold injury, pyogenesis, and nervous diseases. However, no literature discloses that tri-heterocyclic polymethine cyanine dyes promote IFN-γ production by immunocompetent cells. Therefore, the agent comprising a tri-heterocyclic polymethine cyanine dye for promoting IFN-γ production according to the present invention is novel.

### BEST MODE FOR CARRYING OUT THE INVENTION

Following explains the preferred embodiments according to the present invention. As described above, the present invention relates to an agent for promoting IFN-γ production comprising a tri-heterocyclic polymethine cyanine dye. The term "tri-heterocyclic polymethine cyanine dye(s)" as referred to as in the present invention means any of the organic dye compounds having a polymethine chain such as a trimethine chain, pentamethine chain, and heptamethine chain, bound with three heterocyclic groups having the different or same with a thiazole structure or quinoline structure at both ends and the meso position in the polymethine chain. The present invention, as mentioned above, was made based on the original discovery that tri-heterocyclic pentamethine cyanine dyes promote IFN-γ production by immunocompetent cells. Therefore, the tri-heterocyclic polymethine cyanine dyes with either structure (it may be simply described as "cyanine dyes", hereinafter) can be advantageously used in the present invention as long as they are capable of significantly promoting IFN-γ production *in vivo* or *in vitro*.

The compounds represented by the General Formulae 1 and 2 are examples of such cyanine dyes.

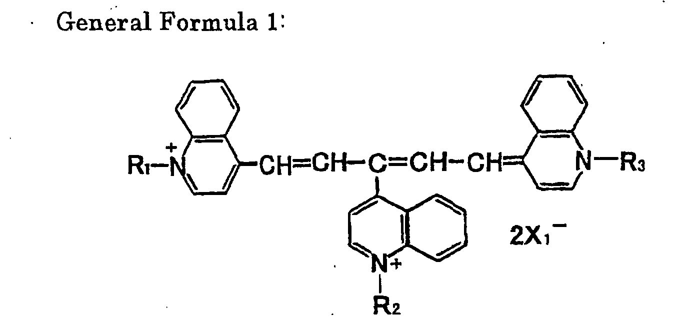

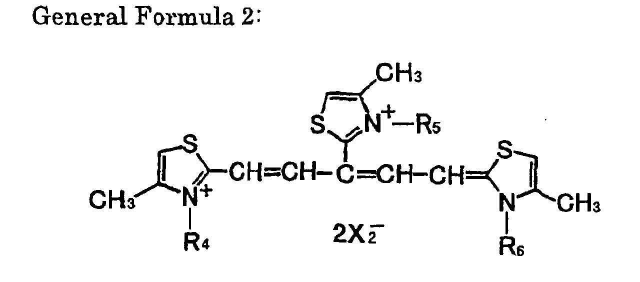

R₁, R₂ and R₃ in the General Formula 1 are the same or different aliphatic carbonyl hydrogen groups, for example, straight or branched linear short chain aliphatic hydrocarbon groups having one to five carbon atoms, such as methyl, ethyl, propyl, isopropyl, butyl, isobutyl, sec-butyl, tert-butyl, pentyl, isopentyl, neopentyl, and tert-pentyl groups. Among the cyanine dyes, those where R₁, R₂, and R₃ are ethyl, methyl or propyl group are particularly preferable because they can be prepared at a low cost and have satisfactory affinity to living bodies.

R₄, R₅ and R₆ in the General Formula 2 are the same or different aliphatic carbonyl hydrogen groups, for example, straight or branched linear middle chain aliphatic hydrocarbon groups having four to ten carbon atoms, such as propyl, isopropyl, pentyl, isopentyl, neopentyl, tert-pentyl, 1-methylpentyl, 2-methylpentyl, hexyl, isohexyl, 5-methyhexyl, heptyl, octyl, nonyl, and decyl groups. Among the cyanine dyes, those where R₄, R₅ and R₆ are straight hexyl, heptyl, or octyl group are particularly preferable because they can be prepared at a low cost and have satisfactory affinity to living bodies.

X₁⁻ and X₂⁻ in the General Formulae 1 and 2 mean physiologically acceptable anions, for example, usually, halogen anion such as fluorine ion, chlorine ion, bromine ion, iodine ion, and perchlorate ion; inorganic acid anions such as sulfate ion, nitrate ion, and phosphate ion; organic acid anions such as acetate ion, propionate ion, benzoate ion, *p*-toluenesulfonate ion, benzenesulfonate ion, nicotinate ion, and orotate ion; more preferably, halogen anions such as fluorine ion, chlorine ion, bromine ion, and iodine ion, most preferably, iodine ion can be mentioned.

Specific examples of the cyanine dyes usable in the present invention are those represented by Chemical Formulae 1 and 2. Since these compounds promote IFN-γ production constantly and do not have toxicity and serious side effects against living bodies, they are extremely useful in the present invention.

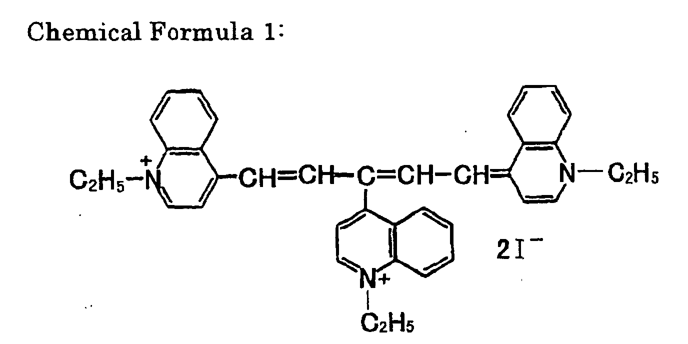

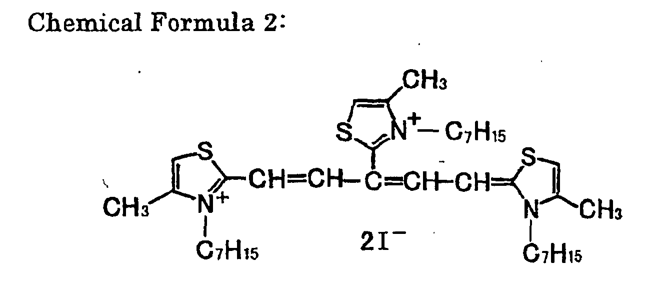

The above cyanine dyes can be obtained in a desired amount with conventional methods or in accordance therewith. For example, the cyanine dyes represented by Chemical Formulae 1 and 2 can be prepared by the method described in "KANKO-SHIKISO (PHOTOSENSITIZING DYE)", edited by Hayami M., published by Sangyo Tosho Publishing Co., Ltd., Tokyo, Japan, pp. 24-30, October 17, 1997, or in accordance therewith. Commercialized cyanine dyes can be also used after appropriately purified. "NK-4" and "NK-19" produced by Hayashibara Biochemical Laboratories, Inc., Okayama, Japan, can be used as such commercialized cyanine dyes.

The use of the agent for promoting IFN-γ production of the present invention is explained as follows. As described above, the cyanine dyes used in the present invention promotes IFN-γ production. Therefore, the agents can be advantageously used as an agent for promoting IFN-γ production in conventional cell culture method for producing IFN-γ, i.e., by treating cultured immunocompetent cells with such agent due to the activity of the cyanine dyes. Also it can be advantageously used as an agent for treating/preventing various IFN-γ susceptive diseases because it does not cause toxicity and serious side effects, even when directly applied to living bodies.

The method of producing IFN-γ using the agent of the present invention comprises a step of allowing an effective amount of the agent to act on immunocompetent cells. Concretely, blood cells including leukocytes or lymphocytes, composing peripheral blood or lymph, as immunocompetent cells; or established immunocompetent cell lines such as KG-1 cell (ATCC CCL-246), Mo cell (ATCC CRL-8066), Jurkat cell (ATCC TIB-52), HuT78 cell (ATCC TIB-161), and EL4 cell (ATCC TIB-39) are suspended in conventional culture medium pre-warmed at a temperature of 30-40°C, which contains 1-10,000 µg/ml, preferably, 10-1,000 µg/ml of the agent of the present invention. Optionally, to the medium can be added a T-cell stimulating substance such as a mitogen, interleukin 2, interleukin 12, or anti-CD3 antibody. The cells are cultured according to a usual manner in the art for 1-100 hours while replacing the culture medium with fresh one at appropriate time intervals and keeping at 30-40°C and pH 5 to 8.

The produced IFN-γ can be purified by conventional methods used for purifying other physiologically active proteins, for example, dialysis, salting out, decanting, filtration, concentration, separatory precipitation, gel filtration chromatography, ion-exchange chromatography, hydrophobic chromatography, affinity chromatography, chromatofocusing, gel electrophoresis, and electrofocusing, which can be applied alone or in combination.

The agent for treating IFN-γ susceptive diseases can be used by directly applying an effective amount of the agent to living bodies. The term "IFN-γ susceptive diseases" as referred to as in the present invention means diseases that IFN-γ is directly or indirectly effective against a source of the disease and capable of treating and/or preventing and includes, for example, viral diseases such as hepatitis, stomatitis, herpes virosis, condyloma acuminatum, acquired immune deficiency syndrome (AIDS); bacterial infectious diseases such as *Candida*, malaria, drug resistant tuberculosis, and atypical mycobacteriosis; malignant tumors such as skin carcinoma, renal cell carcinoma, lung small cell carcinoma, mycosis fungoides, malignant melanoma, chronic granuloma, and neuloblastoma; blood cell cancers such as adult T-cell leukemia, chronic myelogenous leukemia, and malignant lymphoma; immunological diseases such as atopic dermatitis and rheumatoid arthritis; and other diseases such as cheloid, marble bone disease, and superoxide dismutase deficiency.

The agent for promoting IFN-γ production of the present invention has various uses for treating/preventing IFN-γ susceptive diseases as an anti-tumor agent, anti-viral agent, anti-infectious agent, or anti-immunological disease agent. Varying depending on the kinds of cyanine dyes and IFN-γ susceptive diseases, the agent of the present invention contains a cyanine dye(s), usually, 0.005%(w/w) or more, preferably, 0.01 to 1%(w/w) in terms of administration route, and it can be formed into an extract, elixir, inhalant for lower respiratory tract use, capsule, granule, ophthalmic sustained-releasing agent, pill, ophthalmic ointment, oral mucosal adhesive preparation, suspension, emulsion, plaster, suppository, powder, spiritus, tablet, syrup, spreader, apozem, injection, tincture, collyrium, eardrop, collunarium, troche, ointment, cataplasm, pastille, nose air spray, embrocation, limonade, fluidextract, and lotion.

The agent for promoting IFN-γ production of the present invention usually comprises a pharmaceutical agent other than the cyanine dyes as an effective ingredient when used as an agent for treating IFN-γ susceptive diseases, such as excipients, ointment bases, resolvents, corrigents, colorings, and emulsifiers. One or more of dermatologic preparations such as dermatologic bactericides, dermatologic disinfectants, wound protectants, and antiphlogistics; vitamin preparations such as vitamin A, vitamin B, vitamin C, vitamin D, vitamin E, and vitamin K; revitalizers such as calcium preparations, inorganic substance preparations, saccharide preparations, organic acid preparations, protein preparations, amino acid preparations, and organ preparations; cell activation drugs such as chlorophyll preparations and pigment preparations; antitumor drugs such as alkylating agent, antimetabolites, antineoplastic antibiotics, and antitumor plant extract; allergy medications such as antihistamine; chemotherapeutics such as antituberculotics; hormone preparations; antibiotics; biologicals, can be used in the agent as long as they do not prevent the object of the present invention.

The agent for treating IFN-γ susceptive diseases of the present invention further includes pharmaceuticals in the form of a single dose unit form. Such pharmaceuticals comprise the cyanine dye(s), for example, in a content corresponding to multiples (up to fourfold) or divisors (not less than 1/40) of its single dosage, and in a physically united formula suitable for administration. The formulae of such pharmaceuticals include capsules, granules, pills, ophthalmic ointments, powders, tablets, injections, and cataplasms.

The agent of the present invention can be applied to patients depending on the symptom of patients before and after treatment, for example, at a dose of 1 to 10,000 µg/kg a day, preferably, 10 to 1,000 µg/kg a day at once or optionally in a divisional manner, at a frequency of one to seven shots a day over one week to half a year through oral or parenteral route such as intracutaneous, subcutaneous, intramuscular, and intravenous, intranasal, intraperitoneal, or intrarectal route. Oral administration with no injection is preferably used because it accompanies no pain and saves the patients going to hospital.

As known well, IFN-γ relates to biophylaxis system of mammals including humans through phylactic against viruses and bacteria, inhibition of tumor cell proliferation, and regulation of immune functions. As described above, IFN-γ has been practically used and being almost confirmed about its applicable diseases, dose, use, and safety. The compounds, represented by Chemical Formulae 1 and 2 as the typical cyanine dyes of the present invention, have been used for treating/preventing anemia, anorexia, weak constitution, malaise, eczema, wound, burn injury, cold injury, purulent disease, and neurosis without pointing out toxicity or serious side effects for many years. These facts demonstrate that the agent of the present invention as a pharmaceutical can be safely applied to living bodies.

The following explains the effect of the cyanine dyes on INF-γ production by immunocompetent cells based on *ex vivo* experiment using mice as an experimental animal.

### Experiment:

### Effect of cyanine dye on IFN-γ production

Twenty-five of 10-week-old BALB/c female mice were divided into five groups. A powder consisting of one part by weight of "NK-4", a cyanine dye represented by Chemical Formula 1 produced by Hayashibara Biochemical Laboratories, Inc., Okayama, Japan, and 200 parts by weight of sodium bicarbonate was prepared. Then, the mixture was dissolved in physiological saline to give a concentration of 20 mg/ml. Successively, the solution was diluted to give different concentrations as indicated in Table 1 with 20 mg/ml of sodium bicarbonate aqueous solution for administration to the mice. The dilutions were orally administered to the mice in four groups once a day over three days.

Spleens were respectively extracted from the mice in each group, minced and suspended in a usual manner in the art to give the spleen cell suspension. The cell suspensions were collected and diluted in RPMI medium (pH 7.2), supplemented with 10%(v/v) calf serum albumin and containing 10 mM HEPES (N-2-hydroxyethylpiperadine-N'-2-ethanesulfonic acid) and 5 µg/ml lipopolysaccharide, to give a concentration of 1×10⁶ cells/ml, and were cultured at 37°C for 72 hours. IFN-γ was measured by conventional enzyme-linked immunosorbent assay (ELISA). As a control, the mice of the group with no cyanine dye were treated similarly as above. The results are in Table 1. The measured values of IFN-γ were converted into international units (IU) with reference to the standard mouse IFN-γ (Gg02-901-533) obtained form National institute of Health, U.S.A., as an IFN-γ standard.

**Table 1:**

| Dose of Cyanine Dye (µg/kg body weight) | IFN-γ Production (IU/ml) |
|---|---|
| 0 | 0.4 |
| 1 | 1.2 |
| 10 | 2.2 |
| 100 | 2.3 |
| 1,000 | 2.8 |

As the result in Table 1, the control group produced only 0.4 IU/ml of IFN-γ, but, in contrast, the groups orally administered with the cyanine dye showed a remarkable increase of IFN-γ production depending on the increase of the dose. Especially, the group administered with 1,000 µg/kg body weight of the cyanine dye exhibited a high level of IFN-γ production (2.8 IU/ml, corresponding to sevenfold of that of the control group). In addition, all the mice administered with the cyanine dye did not die and had been in good conditions of hair complexion, appetite, and motility. These experimental results revealed that the agent of the present invention promotes IFN-γ production by immunocompetent cells significantly, and does not cause toxicity and serious side effects, even when directly applied to living bodies. Also, the cyanine dye represented by Chemical Formula 1 did not cause any serious side effect such as symptom of poisoning when directly applied to living bodies. LD₅₀ of the cyanine dye represented by Chemical Formula 2 for oral administration, is 1.5 g/kg body weight, and that for intraperitoneal administration is 54 mg/kg body weight.

The following examples explain the agent of the present invention:

### Example 1

### Liquid agent

Either of "NK-4" and "NK-19", cyanine dyes represented by Chemical Formulae 1 and 2 respectively, produced by Hayashibara Biological Laboratories, Inc., Okayama, Japan, was dissolved in physiological saline to give a concentration of 0.01%(w/w). After sterilized by filtrating according to a usual microfiltration method, two kinds of liquid agents were prepared.

The products are stable and useful as an injection, collyrium, or collunarium for treating/preventing malignant tumors, viral diseases, bacterial infectious diseases, and immunological diseases.

### Example 2

### Dried injection

Either of "NK-4" and "NK-19", cyanine dyes represented by Chemical Formulae 1 and 2 respectively, produced by Hayashibara Biological Laboratories, Inc., Okayama, Japan, was dissolved in physiological saline to give a concentration of 0.1%(w/w). After sterilized by filtrating according to a usual microfiltration method, each solution was distributed by 1ml into vials and lyophilized, followed by sealing the vials. Two kinds of liquid agents were prepared.

The products are stable, and are useful as a dried injection for curing and preventing malignant tumors, viral diseases, bacterial infectious diseases, and immunological diseases.

### Example 3

### Ointment

"Hi-Bis Wako", a carboxyvinyl polymer produced by Wako Pure Chemical Industries, Ltd., Osaka, Japan, and "TREHA®", a highly-purified trehalose commercialized by Hayashibara Shoji, Inc., Okayama, Japan free from pyrogen were dissolved in sterilized-distilled water to give respective concentrations of 1.4%(w/w) and 2.0%(w/w). The resulting solution was mixed to homogeneity with either "NK-4" or "NK-19", cyanine dyes represented by Chemical Formula 1 and 2 respectively, produced by Hayashibara Biological Laboratories, Inc., Okayama, Japan, and then adjusted to pH 7.2. Thus, two types of paste containing about 1mg of cyanine dye per 1g of the paste were obtained.

The products are stable, and useful as an ointment for treating/preventing malignant tumors, viral diseases, bacterial infectious diseases, and immunological diseases.

### Example 4

### Tablets

"FINETOSE®", a pulverized anhydrous maltose commercialized by Hayashibara Shoji, Inc., Okayama, Japan, was mixed to homogeneity with either "NK-4" or "NK-19", cyanine dyes represented by Chemical Formula 1 and 2 respectively, produced by Hayashibara Biological Laboratories, Inc., Okayama, Japan. These mixtures were made into tablets in a usual manner to obtain two types of tablets (about 200 mg each), containing about 50 µg of either of the cyanine dyes were obtained.

The products are excellently ingestible and stable and are useful as tablets for treating/preventing malignant tumors, viral diseases, bacterial infectious diseases, and immunological diseases.

### INDUCTRIAL APPLICABILITY

As described above, the present invention was established on the basis of the finding of a novel physiological action of cyanine dyes. Since the agent of the present invention constantly promotes IFN-γ production, it is useful as an agent for promoting IFN-γ production in conventional cell culture method for producing IFN-γ. Further, the agent is also useful as an agent for treating/preventing IFN-γ susceptive diseases related to IFN-γ directly or indirectly, such as viral infectious diseases, bacterial infectious diseases, malignant tumors, and immunological diseases because the agent does not have toxicity and serious side effects even when directly applied to living bodies.

The present invention having such outstanding effects is a significant invention that will greatly contribute to this art.

## Claims

1. An agent for promoting interferon-γ production, which comprises a tri-heterocyclic polymethine cyanine dye.

2. The agent of claim 1, wherein said tri-heterocyclic polymethine cyanine dye is represented by the General Formula 1 or 2: 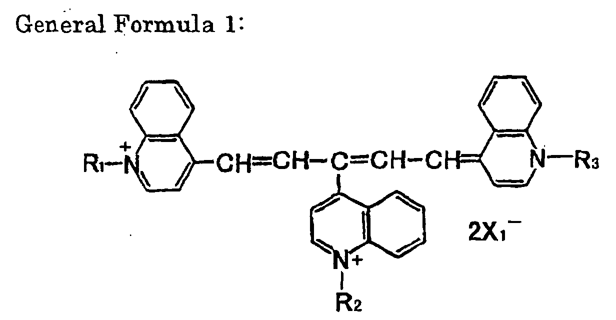 where R₁, R₂ and R₃ are the same or different aliphatic hydrocarbon groups, and X₁⁻ is a physiologically acceptable anion; 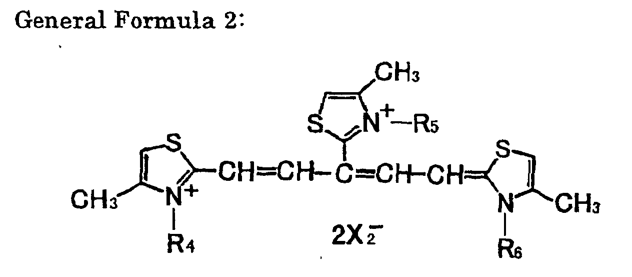 where R₄, R₅ and R₆ are the same or different aliphatic hydrocarbon groups, and X₂⁻ is a physiologically acceptable anion.

3. The agent of claim 1 or 2, which is used for treating interferon-γ susceptive diseases.

4. The agent of any one of claims 1 to 3, which is orally administered to living bodies.

5. A method for producing interferon-γ, which comprises a step of allowing the agent of any one of claims 1 to 4 to act on immunocompetent cells.
